(19) Europäisches Patentamt
European Patent Office
Office européen des brevets

(11) Veröffentlichungsnummer: **0 281 854 B1**

(12) # EUROPÄISCHE PATENTSCHRIFT

(45) Veröffentlichungstag der Patentschrift: **11.12.91**

(51) Int. Cl.$^5$: **C07D 263/34**, G03G 5/06, C07D 413/00

(21) Anmeldenummer: **88102773.4**

(22) Anmeldetag: **25.02.88**

(54) **Neue 4-Chloroxazolverbindungen, Verfahren zu ihrer Herstellung und ihre Verwendung.**

(30) Priorität: **04.03.87 DE 3706881**

(43) Veröffentlichungstag der Anmeldung:
**14.09.88 Patentblatt 88/37**

(45) Bekanntmachung des Hinweises auf die Patenterteilung:
**11.12.91 Patentblatt 91/50**

(84) Benannte Vertragsstaaten:
**DE FR GB**

(56) Entgegenhaltungen:
**EP-A- 0 010 652**
**EP-A- 0 041 674**
**EP-A- 0 041 675**

(73) Patentinhaber: **HOECHST AKTIENGESELLSCHAFT**
**Postfach 80 03 20**
**W-6230 Frankfurt am Main 80(DE)**

(72) Erfinder: **Wingen, Rainer, Dr. Dipl.-Chem.**
**Rotkäppchenweg 10**
**W-6234 Hattersheim 3(DE)**
Erfinder: **Günther, Dieter, Dr. Dipl.-Chem.**
**Nachtigalenweg 1a**
**W-6233 Kelkheim(DE)**
Erfinder: **Lingnau, Jürgen, Dr. Dipl.-Chem.**
**Karolingerstrasse 10**
**W-6500 Mainz-Laubenheim(DE)**

**EP 0 281 854 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Hydrazin-Derivate des 4-Chlor-oxazols, Verfahren zu ihrer Herstellung und ihre Verwendung.

4-Chloroxazole und ihre Verwendung als photoleitfähige Substanzen und als optische Aufheller sind in der EP-B-0 010 652 beschrieben. Ihre Herstellung erfolgt durch Kondensation eines Acylcyanids mit einem Aldehyd in Gegenwart von Chlorwasserstoff in einem etherischen Lösungsmittel.

4-Chloroxazole, die in 2-Position eine Stickstoff haltige Gruppierung vom Hydrazon- oder Pyrazolintyp besitzen, lassen sich jedoch auf diese Weise nicht herstellen, da die benötigten Ausgangsstoffe sich der Reaktion durch Bildung etherunlöslicher Hydrochloride entziehen.

Gegenstand der Erfindung sind neue Hydrazin-Derivate des 4-Chloroxazols der allgemeinen Formel I

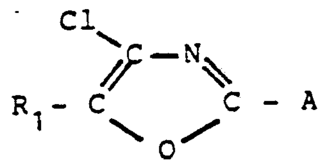

(I),

worin A eine der folgenden Strukturen besitzt

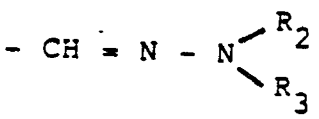 oder 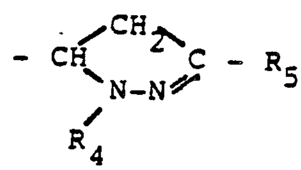

und

| | |
|---|---|
| $R_1$ | Phenyl, das ein- oder mehrfach durch $(C_1$-$C_4)$-Alkoxyreste oder durch Halogen substituiert sein kann, |
| $R_2$,$R_3$ | $(C_1$-$C_6)$-Alkyl oder Phenyl, das durch $(C_1$-$C_4)$-Alkoxyreste oder durch Halogen substituiert sein kann oder |
| $R_2$ und $R_3$ | zusammen eine $-(CH_2)_n-$ Gruppierung mit |
| n | gleich 4 bis 6 bilden, und |
| $R_4$ | henyl, das ein- oder mehrfach durch $(C_1$-$C_4)$-Alkoxyreste oder durch Halogen substituiert sein kann, und |
| $R_5$ | Phenyl, das ein- oder mehrfach durch $(C_1$-$C_4)$-Alkoxy-, Di-$(C_1$-$C_4)$-alkylamino-, Pyrrolidin-1-yl-, Piperidin-1-yl- oder Morpholin-4-yl-reste substituiert sein kann, |

bedeuten. Bevorzugt sind solche Verbindungen, bei denen in der allgemeinen Formel I

A 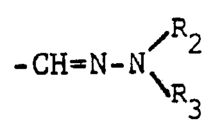

| | |
|---|---|
| $R_1$ | $(C_1$-$C_4)$-Alkoxy-phenyl und |
| $R_2$ und $R_3$ | Phenyl |

bedeuten, oder solche Verbindungen bei denen in der allgemeinen Formel I

A 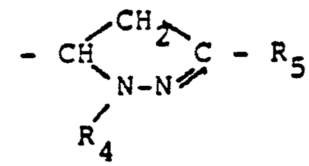

| | |
|---|---|
| $R_1$ | Phenyl, das durch $(C_1-C_4)$-Alkoxy-reste substitiert sein kann, und |
| $R_4$ und $R_5$ | Phenyl |

bedeuten.

Gegenstand der Erfindung sind auch Verfahren zur Herstellung der erfindungsgemäßen 4-Chloroxazole nach der allgemeinen Formel I. Die Verfahren sind dadurch gekennzeichnet, daß man ein Carbonylgruppen haltiges Derivat des 4-Chloroxazols in einem inerten Lösungsmittel in Gegenwart überschüssiger Base bei Temperaturen im Bereich von etwa 60 - 100 °C mit einem substituierten Hydrazin umsetzt und das Reaktionsprodukt isoliert und reinigt. Ein Verfahren ist insbesondere dadurch gekennzeichnet, daß das Carbonylgruppen haltige Derivat des 4-Chloroxazols ein 4-Chloroxazol-2-carbaldehyd mit $R_1$ in der angegebenen Bedeutung ist, das man mit einem Hydrazin mit den für $R_2$ und $R_3$ angegebenen Bedeutungen in Gegenwart überschüssiger Base bei einer Temperatur im Bereich von 60 - 80 °C in einem inerten Lösungsmittel umsetzt und das Reaktionsprodukt isoliert und reinigt. Dabei kann man das Reaktionsprodukt in der Kälte isolieren und chromatographisch oder durch Umkristallieren reinigen. Ein weiteres Verfahren ist dadurch gekennzeichnet, daß das Carbonylgruppen haltige Derivat des 4-Chloroxazols ein $\alpha$, $\beta$-ungesättigtes Keton mit der für $R_5$ angegebenen Bedeutung ist, das man mit einem substituierten Hydrazin, dessen Substituent die für $R_4$ angegebene Bedeutung hat, in Gegenwart überschüssiger Base bei einer Temperatur im Bereich von 70 - 100 °C in einem inerten Lösungsmittel umsetzt und das Reaktionsprodukt isoliert und reinigt.

Wie in dem beigefügten Schema 1 angegeben, können die erfindungsgemäßen Verbindungen mit Hydrazinstruktur aus dem 4-Chloroxazol-2-carbaldehyd II und einem Hydrazin III mit den für $R_2$ und $R_3$ angebenen Bedeutungen erhalten werden, indem man die Komponenten in einem niederen Alkohol wie Methanol, Ethanol oder Propanol auf Temperaturen im Bereich von etwa 60 - 80 °C, vorzugsweise 70 - 75 °C über mehrere Stunden erhitzt, das Rohprodukt ausfällt, zum Beispiel durch Auskristallisation, und nach Trocknen eine Reinigung des Rohproduktes vornimmt, die durch Chromatographie oder durch Umkristallisation erfolgen kann.

Die erfindungsgemäßen Verbindungen mit einer Pyrazolinstruktur sind auf dem in Schema 2 angeführten Weg zugänglich.

Die Reaktion des 4-Chloroxazol-2-carbaldehyds II mit einem Keton IV, das die für $R_5$ angegebene Bedeutung hat, erfolgt in an sich literaturbekannter Weise. Die Umsetzung erfolgt vorzugsweise in der Schmelze bei Temperaturen im Bereich zwischen etwa 80 - 120 °C, vorzugsweise bei etwa 100 °C, in Gegenwart von 1 Molequivalent Zinkchlorid. Das anfallende Gemisch von $\alpha,\beta$-ungesättigtem Keton V und $\beta$-Hydroxy-Keton wird vorzugsweise nicht getrennt, sondern die Hydroxyverbindung durch Wasserabspaltung, zum Beispiel unter Säure-Katalyse in siedendem Toluol, in das $\alpha,\beta$-ungesättigte Keton überführt. Man isoliert das Keton aus dem Reaktionsgemisch wie zum Beispiel durch Auskristallisieren. Das erhaltene Keton V wird mit einem Hydrazin VI mit der für $R_4$ angegebenen Bedeutung in einem inerten organischen Lösungsmittel in Gegenwart überschüssiger Base bei einer Temperatur im Bereich von 70 - 100 °C, vorzugsweise 80 °C zur Reaktion gebracht und das erhaltene Reaktionsprodukt isoliert und gereinigt. Als Lösungsmittel dient vorzugsweise Ethanol, als Base Kaliumhydroxid und die Reinigung erfolgt chromatographisch oder durch Umkristallisieren.

Wie in Schema 3 angegeben, werden die 4-Chloroxazol-2-carbaldehyde II durch Reduktion der entsprechenden Säurechloride X mittels Wasserstoff unter Palladium-Katalyse hergestellt. Ausgangsmaterial für die Säurechloride sind die entsprechenden Carbonsäureester IX, aus denen sie in literaturbekannter Weise durch Hydrolyse und Reaktion mit einem Chlorierungs-mittel erhalten werden können. Die entsprechenden Ester kann man aus den Acylcyaniden VII und den Glyoxylsäureestern VIII in Gegenwart von Chlorwasserstoff erhalten.

Von besonderem Interesse sind die erfindungsgemäßen Hydrazinderivate des 4-Chloroxazols der allgemeinen Formel I als photoleitfähige Substanzen, beispielsweise in elektrophotographischen Aufzeichnungsmaterialien. Solche bestehen aus einer leitfähigen Unterlage und einem Schichtaufbau, der im Dunkeln isolierend ist, aber unter Belichtung leitend wird. Der Aufbau kann aus einer oder mehreren Schichten bestehen. Im Fall einer einzigen Schicht ist mindestens eine photoleitfähige Substanz in mindestens einem Bindemittel gelöst oder dispergiert auf eine leitende Unterlage aufgebracht. Ein mehrschichtiger Aufbau besteht aus mindestens einer ladungsträgererzeugenden Schicht und mindestens einer ladungstransportierenden Schicht.

Die Erzeugung von Ladungsbildern auf elektrophotographischen Schichten erfolgt nach der Aufladung durch einen Belichtungsschritt. Durch Belichtung wird zunächst elektrische Ladung erzeugt, die in einem weiteren Schritt durch die Schicht hindurch bis an die Schichtoberfläche transportiert wird und dort vorhandene Ladung neutralisiert. Dabei unterscheidet man Photorezeptoren, bei denen Ladungserzeugung und Ladungstransport durch die gleiche chemische Substanz bewirkt werden, von solchen, bei denen die Ladungserzeugung durch Zugabe einer zweiten Substanz, des Sensibilisators, erreicht wird. Dadurch wird die Möglichkeit gegeben, Elektrophotographie auch mit Licht einer Wellenlänge zu betreiben, das von der eigentlich photoleitfähigen Substanz nicht absorbiert wird.

Zur homogenen Sensibilisierung werden die in den Farbstofftabellen von Schultz (7. Auflage, 1. Band, 1931) aufgeführten organischen Farbstoffe eingesetzt, zum Beispiel Triarylmethanfarbstoffe, wie Brillantgrün (Nr. 760, S. 314), Victoriablau B (Nr. 822, S. 347), Methylviolett (Nr. 783, S. 327), Kristallviolett (Nr. 785, S. 329), Säureviolett 6B (Nr. 381, S. 351); Xanthenfarbstoffe, und zwar Rhodamine, wie Rhodamin B (Nr. 864, S. 365), Rhodamin 6G (Nr. 866, S. 366), Rhodamin G extra (Nr. 865, S. 366), Sulforhodamin B (Nr. 863, S. 364) und Echtsäureeosin G (Nr. 870, S. 368) sowie Phthaleine, wie Eosin S (Nr. 883, S. 375), Eosin A (Nr. 881, S. 374), Erythrosin (Nr. 866, S. 376), Phloxin (Nr. 890, S. 378), Rose bengale (Nr. 889, S. 378) und Fluorescein (Nr. 880, S. 373); Thiazinfarbstoffe, wie Methylenblau (Nr. 1038, S. 449); Acridinfarbstoffe, wie Acridingelb (Nr. 901, S. 383), Acridinorange (Nr. 908, S. 387) und Trypaflavin (Nr. 906, S. 386); Chinolinfarbstoffe, wie Pinacyanol (Nr. 924, S. 396) und Kryptocyanin (Nr. 927, S. 397); Chinonfarbstoffe und Ketonfarbstoffe, wie Alizarin (Nr. 1141, S. 449), Alizarinrot S (Nr. 1145, S. 502) und Chinalizarin (Nr. 1148, S. 504); Cyaninfarbstoffe (Polymethinfarbstoffe), wie Astrazongelb 3G (Colour Index Nr. (C.I.) 48 055) und 5 G (C.I. 48 065), Basic Yellow 52115 (C.I. 48 060), Astrazongelb GRL, Astrazonorange G (C.I. 48 040) und R (C.I. 48 035), Astrazonorange 3R (noch nicht klassifiziert).

Pyryliumsalze, Thiapyryliumsalze, Benzopyryliumsalze können ebenfalls verwendet werden.

Sensibilisierungsfarbstoffmischungen können ebenfalls vorliegen.

Als dispers verteilte Farbstoffe oder Pigmente können auch metallhaltige oder metallfreie Phthalocyaninpigmente, zum Beispiel Kupferphthalocyanin, Perinon-, Thioindigo-, höher annellierte Chinon-, Chinacridon-, Perylen-, Anthrachinon-, Dioxazin-, Azo-, Bisazo-, Trisazo-, Cyanin-Pigmente oder Benzo(thio)-xanthen-Derivate sowie deren Mischungen eingesetzt werden. Davon sind besonders bevorzugt Phthalocyaninpigmente, wie die verschiedenen Cu-Phthalocyaninmodifikationen ($\alpha$, $\beta$, $\epsilon$), Bis- und Trisazopigmente, Perylen-3,4,9,10-tetracarbonsäureanhydrid und seine Imidderivate, oder (Iso)Violanthrone. Sie können bis zu 30 %, bezogen auf die Gesamtmasse der photoleitfähigen Schicht, vorhanden sein, bevorzugt werden Mengen im Bereich von 0,1 bis 10 % Pigment verwendet.

Das hochisolierende Bindemittel für die ladungsträgererzeugende Schicht und für die Ladungstransportschicht kann gleich oder unterschiedlich sein. Als solche sind hinsichtlich der Flexibilität, der Filmeigenschaften und der Haftfestigkeit Natur- und Kunstharze geeignet. Hierzu gehören Polyesterharze, die Mischpolyester aus Iso- und Terephthalsäure mit Glykolen darstellen. Auch Silikonharze haben sich als geeignet erwiesen. Polycarbonatharze sind gut einsetzbar. Besonders bevorzugt für die Herstellung von Druckformen und gedruckten Schaltungen sind Bindemittel, die in wäßrigen oder alkoholischen Lösungsmittelsystemen, gegebenenfalls unter Säure- oder Alkalizusatz, löslich sind. Aus physiologischen und Sicherheitsgründen scheiden aromatische oder aliphatische, leicht brennbare Lösungsmittel aus. Geeignete Harzbindemittel sind hiernach hochmolekulare Substanzen, die alkalilöslich machende Gruppen tragen. Solche sind beispielsweise Säureanhydrid-, Carboxyl-, Carbonsäureamid-, Phenol-, Sulfonsäure-, Sulfonamid- oder Sulfonimid-Gruppen. Bevorzugt werden Harzbindemittel mit hohen Säurezahlen eingesetzt. Mischpolymerisate mit Anhydridgruppen können mit gutem Erfolg verwendet werden, da durch das Fehlen freier Säuregruppen die Dunkelleitfähigkeit gering ist, trotz guter Alkalilöslichkeit. Besonders bewährt haben sich Copolymerisate aus Styrol und Maleinsäureanhydrid, Sulfonylurethane gemäß DE-OS 32 10 577, und Copolymerisate der Acryl- bzw. Methacrylsäure.

Als übliche Zusätze enthalten die Schichten Substanzen, die der Beschichtungslösung zugesetzt werden und dadurch die Oberflächenstruktur und die Flexibilität verbessern.

Dies können zum Beispiel Weichmacher, wie Triphenylphosphat, oder Verlaufmittel, wie Silikonöle, sein.

Als elektrisch leitende Schichtträger sind Materialien mit genügend elektrisch leitenden Eigenschaften geeignet, wie sie auch bisher bereits zu diesem Zweck verwendet wurden. Der Schichtträger kann in Form eines flexiblen Bandes oder einer Platte vorliegen. In bevorzugter Ausführungsform ist der Schichtträger für die Herstellung von Druckformen und gedruckten Schaltungen geeignet und besteht zum Beispiel aus einer Aluminium-, Zink-, Magnesium-, Kupfer-, Eisen-, Nickel- oder einer Mehrmetallplatte. Es kommen auch

metallisierte, zum Beispiel metallbedampfte Kunststoffolien, wie aluminiumbedampfte Polyesterfolien, oder auch kupferkaschierte Polyimidfolien und -platten in Frage.

Besonders bewährt haben sich oberflächenveredelte Schichtträger aus Aluminium. Die Oberflächenveredlung besteht in einer mechanischen oder elektrochemischen Aufrauhung und gegebenenfalls in einer anschließenden Anodisierung und Behandlung mit Polyvinylphosphonsäure gemäß DE-OS 16 21 478, entsprechend US-PS 4,153,461.

Ganz allgemein kann eine isolierende Zwischenschicht auf dem Schichtträger vorhanden sein, wie eine thermisch, anodisch bzw. chemisch erzeugte Metalloxidschicht, zum Beispiel aus Aluminiumoxid. Diese Sperrschicht hat die Aufgabe, die Ladungsträgerinjektion vom elektrisch leitenden Schichtträger im Dunkeln in die Ladungsträger erzeugende Schicht herabzusetzen bzw. zu verhindern.

Weiterhin wird durch die Sperrschicht die Haftung der folgenden Schichten auf dem Schichtträger günstig beeinflußt. Für organische Sperrschichten können verschiedene Natur-oder Kunstharzbindemittel verwendet werden, die gut auf einer Metall-bzw. Aluminiumoberfläche haften und beim nachfolgenden Anbringen der weiteren Schichten keine An- oder Ablösung erfahren. Die Dicke der organischen Sperrschicht liegt im Bereich von 1 µm, die einer Metalloxidschicht in der Größenordnung von 1o bis $1o^4$ nm.

Zur Herstellung von beispielsweise gedruckten Schaltungen, wie sie in der Elektronik üblich sind, kann die photoleitfähige Schicht auch zunächst auf einen Zwischenträger aufgebracht werden, von dem aus sie als sogenannter Trockenresist auf den Schichtträger anschließend oder später übertragen wird. Dies kann zum Beispiel durch Laminieren erfolgen. Als Zwischenträger haben sich Kunststoffolien, wie solche aus Polyester, insbesondere aus Polyethylenterephthalatfolie, besonders bewährt.

Die Beschichtungen bringt man in üblicher Weise auf, zum Beispiel durch Rakel- oder Sprühantrag. Vorzugsweise wird der Antrag mit einem Fließer vorgenommen. Die Trocknung der Schichten erfolgt beispielsweise in Trockenkanälen, wobei die verschiedenen Trocknungsstufen durch die Temperatur der einzelnen Bereiche, durch die Laufgeschwindigkeit des Materials und durch den herrschenden Luftdurchsatz festgelegt werden.

Die Erfindung wird anhand der folgenden Beispiele für die Herstellungsverfahren und für die Anwendung als photoleitfähige Substanzen näher beschrieben.

Allgemeine Vorschriften zur Herstellung der Verbindungen die als A-Gruppierung

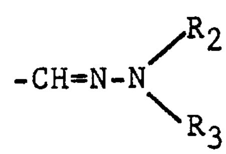

tragen (Tabelle 1):

50 mmol Aldehyd II und 50 mmol Hydrazin-hydrochlorid III werden in 200 ml Ethanol (95%) 2 h auf 80 °C erhitzt. Der nach Erkalten angefallene Feststoff wird an Kieselgel mit Dichlormethan chromatographiert und das erhaltene nur noch schwach gelbe Hydrazon aus Cyclohexan umkristallisiert.

Elementaranalyse für

4-Chlor-5-(4-methoxy)phenyl-oxazol-2-carbaldehyd-N,N-diphenylhydrazon, wobei in der allgemeinen Formel I

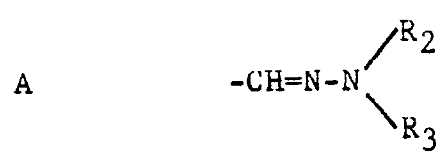

| | |
|---|---|
| $R_1$ | Methoxyphenyl und |
| $R_2$ und $R_3$ | Phenyl bedeuten: |

$C_{23}H_{18}ClN_3O_2$ ber. C 68.4 H 4.5 Cl 8.8 N 10.4
$C_{23}H_{18}ClN_3O_2$ gef. C 68.6 H 4.5 Cl 9.2 N 10.5

Allgemeine Vorschriften zur Herstellung der Verbindungen (Tabelle 1), die als A-Gruppierung

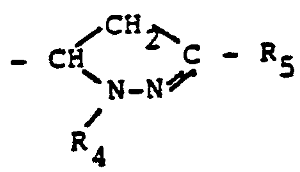

tragen:

20 mmol Keton V, 22 mmol Hydrazin VI und 30 mmol Kaliumhydroxid werden in 200 ml Ethanol (95 %) 3 h auf 80 °C erhitzt. Das Lösungsmittel wird im Vakuum abdestilliert, der Rückstand in Dichlormethan aufgenommen, neutral gewaschen und mit Dichlormethan an Kieselgel chromatographiert. Das Eluat wird eingedampft und der Rückstand aus 2-Propanol umkristallisiert.

Elementaranalyse für

2,5-Phenyl-3-(4-chlor-5-phenyl-oxazol-2-yl)-2,4-dihydro-3(H)-pyrazol, wobei in der allgemeinen Formel I

A 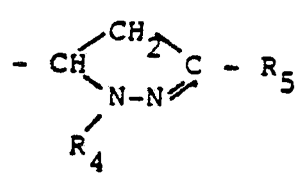

$R_4 = R_5$ Phenyl bedeuten:
$C_{24}H_{18}ClN_3O$ ber. C 72.1 H 4.5 Cl 8.9 N 10.5
$C_{24}H_{18}ClN_3O$ gef. C 71.9 H 4.6 Cl 8.7 N 10.3

Allgemeine Vorschrift zur Herstellung des $\alpha,\beta$- ungesättigten Ketons nach Formel V (Tabelle 2).

100 mmol Aldehyd II, 110 mmol Keton IV und 100 mmol Zinkchlorid werden 1 h auf 100 °C erhitzt. Die erkaltete Schmelze wird in Dichlormethan gelöst, mit 2 n HCl gewaschen und das Lösungsmittel abdestilliert.

Der Rückstand wird in 100 ml Toluol aufgenommen und mit 0,1 g p-Toluolsulfonsäure erhitzt unter azeotroper Entfernung des Reaktionswassers. Der nach Abdampfen des Lösungsmittels erhaltene Rückstand wird aus 2-Propanol umkristallisiert.

Herstellung der 4-Chloroxazol-2-carbaldehyde:

Durch Umsetzung von Benzoylcyanid bzw. Methoxy-benzoylcyanid bzw. 3,4,5-Trimethoxy-benzoylcyanid mit Glyoxylsäureestern in Gegenwart von Chlorwasserstoff in Diethylether bei 0 °C, hydrolytische Aufarbeitung nach Umkristallisation sind die Ester IX erhältlich.

Verseifung von IX mit wäßriger Kaliumhydroxidlösung und Umsetzung des erhaltenen Carbonsäure-K-Salzes bzw. der nach Ansäuern erhaltenen Carbonsäure mit Thionylchlorid ergibt die Säurechloride X, die ohne weitere Reinigung in Toluol bei 70 °C in Gegenwart von 5 Gew.-% $Pd/BaSO_4$ (5%) mit Wasserstoff bis zum Ende der Chlorwasserstoff-Entwicklung begast werden. Nach Abtrennung des Hydrierkatalysators, Abdampfen des Lösungsmittels und Reinigung durch Umkristallisation aus Cyclohexan werden die Aldehyde II erhalten.

Die erhaltenen Verbindungen sind in der beigefügten Tabelle 3 aufgeführt.

Anwendungsbeispiele:

Die Ausprüfung der elektrophotographischen Eigenschaften der erfindungsgemäßen Verbindungen der Formel I erfolgte in unterschiedlichen Schichtrezepturen:

Beschichtungsrezeptur 1:

15.0 g Hostapermorange GR (Pigment Orange 43, C.I. 71.105) wurden in eine Lösung von 10 g Polybutylmethacrylat (® Plexigum P676, Röhm GmbH) in 200 g Tetrahydrofuran (THF) eingetragen und durch Mahlen in einer Kugelmühle während 2 Stunden dispergiert. Nach Zusatz von 32 g Polymethylmethacrylat (® Plexigum M345) in 340 g Tetrahydrofuran wurde die Schicht mit einem Schichtgewicht von 6 g/m$^2$ auf mit Aluminium bedampfte Polyethylenterephthalatfolie aufgetragen und getrocknet. Die getrocknete Schicht wurde dann mit einer 5 %igen Lösung der jeweiligen erfindungsgemäßen Verbindungen in THF behandelt und erneut getrocknet.

Beschichtungsrezeptur 2:

5 g der jeweiligen erfindungsgemäßen Verbindungen, 5 g eines Copolymeren aus Maleinsäurehalbester und Styrol, Zersetzungspunkt 200 bis 240 °C, und 0.05 g Rhodamin B wurden in 90 g Tetrahydrofuran gelöst und so auf einen aufgerauhten und anodisierten Aluminium-Offsetdruckplattenträger aufgebracht, daß ein Trockenschichtgewicht von 6 g/m$^2$ resultierte.

Beschichtungsrezeptur 3:

Auf eine Aluminium bedampfte Polyethylenterephthalatfolie wurde zunächst eine Ladungsträger-erzeugende Schicht aus N,N'-Dimethylperylen-3,4,9,10-tetracarbonsäurediimid in einer Schichtdicke von 200 mg/m$^2$ durch Sublimation aufgetragen. Auf diese Schicht wurde eine Lösung aus 50 Gewichtsteilen der jeweiligen erfindungsgemäßen Substanzen und 50 Gewichtsteilen eines Polyesters (® Dynapol L206, Dynamit Nobel) so aufgetragen, daß ein Schichtgewicht dieser Ladungstransportschicht von 10.5 g/m$^2$ resultierte.

Die Resultate der elektrophotographischen Untersuchungen der gemäß den Beispielen hergestellten Schichten sind in der beigefügten Tabelle 4 zusammengestellt. Dabei bedeuten E2, E4 und E8 die Belichtungsenergien in µJ/cm$^2$, die bei einer Lichtintensität von 3 µW/cm$^2$ aufgebracht werden müssen, um eine Entladung von der ursprünglichen Auflassung Uo auf 0.5 Uo, 0.25 Uo bzw. 0.125 Uo zu bewirken. Die Messungen der Proben gemäß Schichtrezeptur 1 erfolgten bei 485 nm, die der Proben gemäß Schichtrezepturen 2 und 3 bei Weißlicht (Halogen-Wolfram-Lampe, Kantenfilter bei 650 nm). Ue bedeutet die nach 10 Sekunden Belichtung auf der Schicht verbliebene Restladung.

Zum Vergleich wurden die Beschichtungsrezepturen auch unter Verwendung der Verbindungen XIII (DE-PS 10 58 836) bzw. XIV (EP-B- 0 010 652) untersucht. Bei Beschichtungsrezeptur 1 wurde zum Vergleich auch auf die Nachbehandlung mit einer photoleiterhaltigen Lösung verzichtet (o.Ph.).

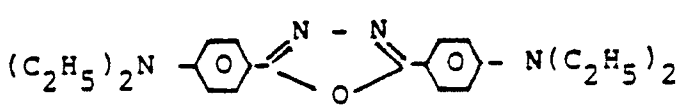

XIII

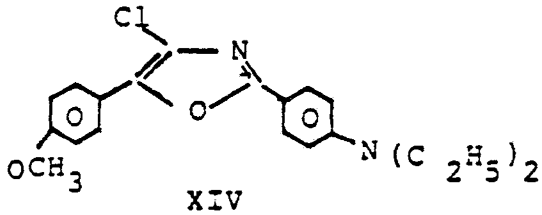

XIV

TABELLE 1

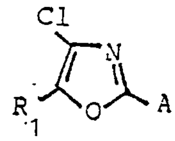 I

| Nr. | A | $R_1$ | $R_2$ | $R_3$ | $R_4$ | $R_5$ | Smp[° C] |
|---|---|---|---|---|---|---|---|
| I a | | 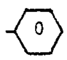 | 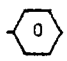 | | - | - | 208-209 |
| b | " | 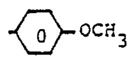 | $-C_2H_5$ | 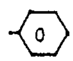 | - | - | 148-149 |
| c | " | 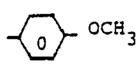 | $-(CH_2)_5-$ | | - | - | 104-106 |
| d | " | 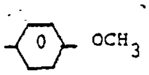 | 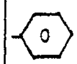 | 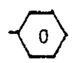 | - | - | 187-189 |
| e | " | 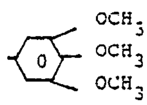 | 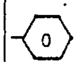 | 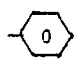 | - | - | 180-181 |
| f | " | 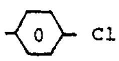 | 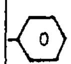 | 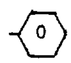 | - | - | 187-188 |
| g | 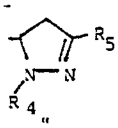 | 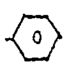 | - | - | 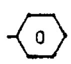 | 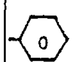 | 148-149 |
| h | " | 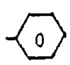 | - | - | 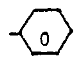 | 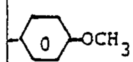 | 155-156 |
| i | " | 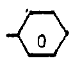 | - | - | 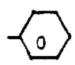 | 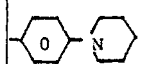 | 198-199 |
| k | " | 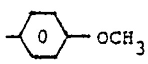 | - | - | 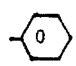 | 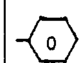 | 143-144 |
| l | " | 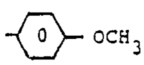 | - | - | 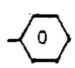 | 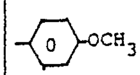 | 152-153 |
| m | " | 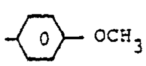 | - | - | 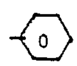 | 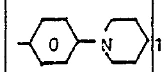 | 173-174 |
| n | " | 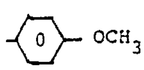 | - | - | 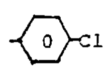 | 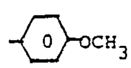 | 171-172 |
| o | " | 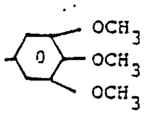 | - | - | 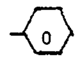 | 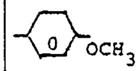 | 156-157 |

TABELLE 2

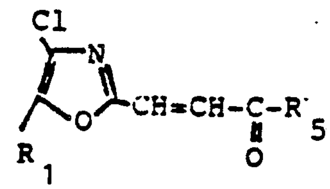

| Nr. | $R_1$ | $R_5$ | Smp. [°C] |
|---|---|---|---|
| V a | 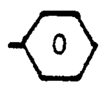 | 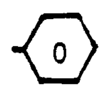 | 129-130 |
| b | 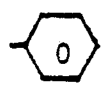 | 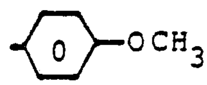 | 153-154 |
| c | 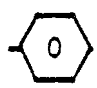 | 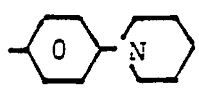 | 132-134 |
| d | 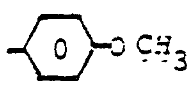 | 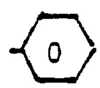 | 155-156 |
| e | 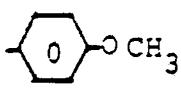 | 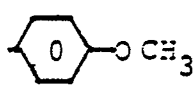 | 134-135 |
| f | 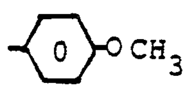 | 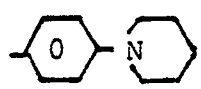 | 160-161 |
| g | 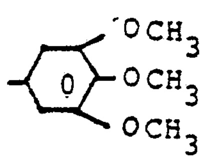 | 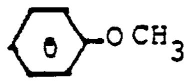 | 143-144 |

TABELLE 3

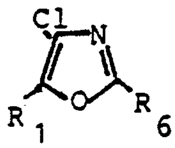

| Nr. | $R_1$ | $R_6$ | Smp. [°C] |
|---|---|---|---|
| IX a | 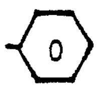 | $-CO_2CH_3$ | 80- 81 |
| b | 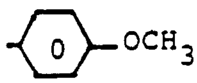 | $-CO_2CH_3$ | 118-119 |
| c | 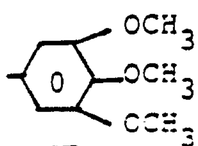 | $-CO_2CH_3$ | 155-156 |
| II a | 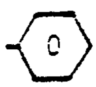 | -CHO | 73-74 |
| b | 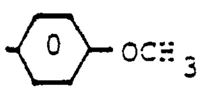 | -CHO | 124-125 |
| c | 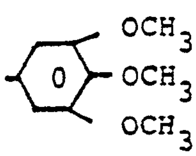 | -CHO | 133-134 |

Tabelle 4

| | Besch. Rez. | -Uo | - Ue | E2 | E4 | E8 |
|---|---|---|---|---|---|---|
| Ib | 1 | 418 | 15 | 5.7 | 7 | 8 |
| Id | 1 | 240 | 7 | 2.4 | 4.2 | 7.8 |
| Ie | 1 | 647 | 67 | 9.3 | 15 | 25 |
| Im | 1 | 592 | 15 | 4.1 | 5.8 | 8.4 |
| In | 1 | 675 | 15 | 5 | 6 | 8 |
| XIII | 1 | 651 | 15 | 4.6 | 6.2 | 8.4 |
| XIV | 1 | 525 | 75 | 20.4 | 62 | |
| o.Ph. | 1 | 746 | 319 | 75.3 | | |
| Ib | 2 | 489 | 181 | 26.6 | | |
| Ie | 2 | 632 | 264 | 30.1 | | |
| If | 2 | 722 | 304 | 40.1 | | |
| Ig | 2 | 734 | 237 | 29.2 | | |
| Ik | 2 | 770 | 734 | | | |
| Il | 2 | 797 | 209 | 25.9 | | |
| Io | 2 | 999 | 896 | | | |
| XIII | 2 | 580 | 31 | 6.8 | 15 | 29 |
| XIV | 2 | 398 | 11 | 10.6 | 30 | 62 |
| Ii | 3 | 272 | 248 | | | |
| Il | 3 | 865 | 39 | 4 | 10 | 20 |
| XIII | 3 | 205 | 7 | 4.9 | 10.1 | 18.4 |

SCHEMATA

Schema 1

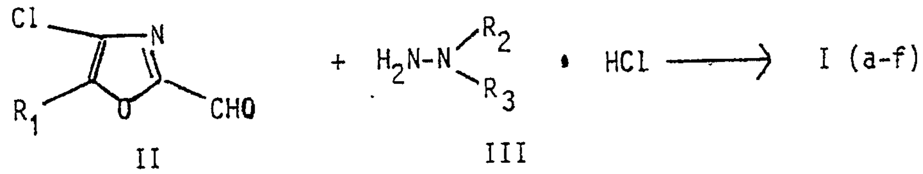


II III

Schema 2

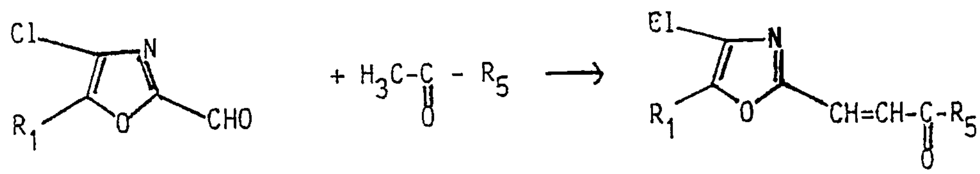

II IV V

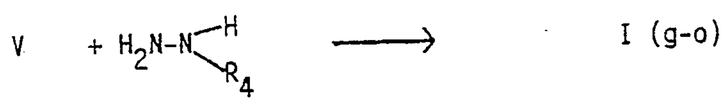


VI

Schema 3

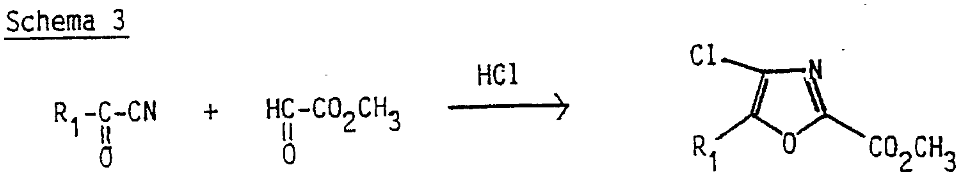


VII VIII IX

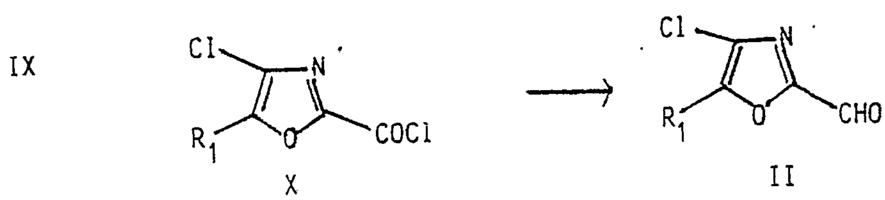


X II

## Patentansprüche

**1.** Hydrazinderivate des 4-Chloroxazols der allgemeinen Formel I

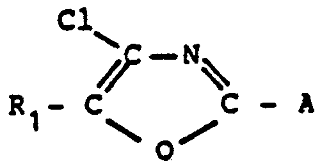

(I),

worin A eine der folgenden Strukturen besitzt

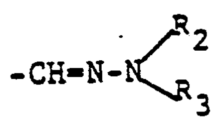

oder

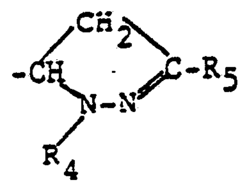

und

| | |
|---|---|
| $R_1$ | Phenyl, das ein- oder mehrfach durch $(C_1-C_4)$-Alkoxyreste oder durch Halogen substituiert sein kann, |
| $R_2$, $R_3$ | $(C_1-C_6)$-Alkyl oder Phenyl, das durch $(C_1-C_4)$-Alkoxyreste oder durch Halogen substituiert ein kann oder |
| $R_2$ und $R_3$ | zusammen eine $-(CH_2)_n-$ Gruppierung mit |
| n | gleich 4 bis 6 bilden, und |
| $R_4$ | Phenyl, das ein- oder mehrfach durch $(C_1-C_4)$-Alkoxyreste oder durch Halogen substituiert sein kann, und |
| $R_5$ | Phenyl, das ein- oder mehrfach durch $(C_1-C_4)$ Alkoxy-, Di-$(C_1-C_4)$-alkylamino-, Pyrrolidin1-yl-, Piperidin-1-yl- oder Morpholin-4-yl-reste substituiert sein kann, |

bedeuten.

2. Verbindungen nach Anspruch 1, worin

A 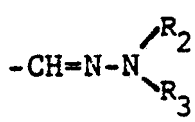

| | |
|---|---|
| $R_1$ | $(C_1-C_4)$-Alkoxy-phenyl und |
| $R_2$ und $R_3$ | Phenyl |

bedeuten.

3. Verbindungen nach Anspruch 1, worin

A 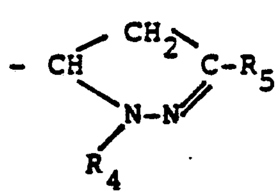

| | |
|---|---|
| $R_1$ | Phenyl, das durch $(C_1-C_4)$-Alkoxy-reste substitiert sein kann, und |
| $R_4$ und R | $_5$ Phenyl |

bedeuten.

4. Verfahren zur Herstellung von Hydrazinderivaten des 4-Chloroxazols gemäß Anspruch 1, dadurch gekennzeichnet, daß man ein Carbonylgruppen haltiges Derivat des 4-Chloroxazols in einem inerten Lösungsmittel in Gegenwart überschüssiger Base bei Temperaturen im Bereich von etwa 60 - 100 °C mit einem substituierten Hydrazin umsetzt und das Reaktionsprodukt isoliert und reinigt.

5. Verfahren nach Anspruch 4, dadurch gekennzeichnet, daß das Carbonylgruppen haltige Derivat des 4-Chloroxazols ein 4-Chloroxazol-2-carbaldehyd mit $R_1$ in der angegebenen Bedeutung ist, das man mit einem Hydrazin mit den für $R_2$ und $R_3$ angegebenen Bedeutungen in Gegenwart überschüssiger Base bei einer Temperatur im Bereich von 60 - 80 °C in einem inerten Lösungsmittel umsetzt und das Reaktionsprodukt isoliert und reinigt.

6. Verfahren nach Anspruch 4, dadurch gekennzeichnet, daß das Carbonylgruppen haltige Derivat des 4-Chloroxazols ein α, β-ungesättigtes Keton mit der für $R_5$ angegebenen Bedeutung ist, das man mit einem substituierten Hydrazin, dessen Substituent die für $R_4$ angegebene Bedeutung hat, in Gegenwart überschüssiger Base bei einer Temperatur im Bereich von 70 - 100 °C in einem inerten Lösungsmittel umsetzt und das Reaktionsprodukt isoliert und reinigt.

7. Verwendung der Verbindungen der allgemeinen Formel I nach Anspruch 1 als photoleitfähige Substanzen.

## Claims

1. Hydrazine derivatives of 4-chlorooxazole of the general formula I

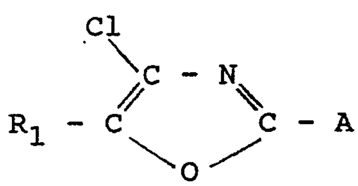

(I),

wherein

A has one of the following structures:

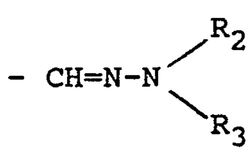

or

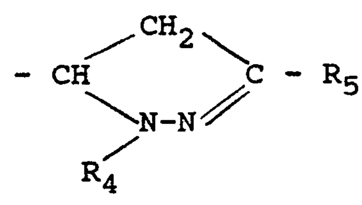

and

$R_1$ denotes phenyl which may be substituted in one or more positions by $(C_1-C_4)$-alkoxy radicals or by halogen,

$R_2$,$R_3$ denote $(C_1-C_6)$-alkyl or phenyl which may be substituted by $(C_1-C_4)$-alkoxy radicals or by halogen or

$R_2$ and $R_3$ together form a $-(CH_2)_n$ grouping, in which

n = 4 to 6, and

$R_4$ denotes phenyl which may be subtituted in one or more positions by $(C_1-C_4)$-alkoxy radicals or by halogen, and

$R_5$ denotes phenyl which may be substituted in one or more positions by $(C_1-C_4)$-alkoxy, di-$(C_1-C_4)$-alkylamino, pyrrolidin-1-yl, piperidin-1-yl or morpholin-4-yl, radicals.

2. Compounds as claimed in claim 1, wherein

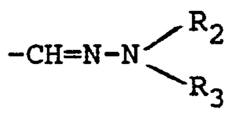

| | |
|---|---|
| A | denotes |
| $R_1$ | denotes $(C_1$-$C_4)$-alkoxy-phenyl and |
| $R_2$ and $R_3$ | denote phenyl. |

3. Compounds as claimed in claim 1, wherein

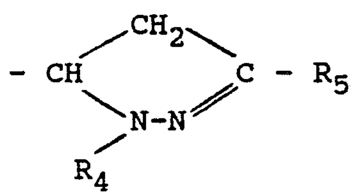

| | |
|---|---|
| A | denotes |
| $R_1$ | denotes phenyl which may be substituted by $(C_1$-$C_4)$-alkoxy radicals and |
| $R_4$ and $R_5$ | denote phenyl. |

4. A process for the preparation of hydrazine derivatives of 4-chlorooxazole as claimed in claim 1, wherein a carbonyl-group-containing derivative of 4-chlorooxazole is reacted with a substituted hydrazine in an inert solvent in the presence of an excess base, at temperatures in the range from about 60 °C to 100 °C, and the reaction product is isolated and purified.

5. A process as claimed in claim 4, wherein the carbonyl-group-containing derivative of 4-chlorooxazole is a 4-chlorooxazole-2-carbaldehyde, in which $R_1$ has the indicated meaning, which is reacted with a hydrazine, in which $R_2$ and $R_3$ have the indicated meanings, in the presence of an excess base, at a temperature in the range from 60 °C to 80 °C, in an inert solvent, and the reaction product is isolated and purified.

6. A process as claimed in claim 4, wherein the carbonyl-group-containing derivative of 4-chlorooxazole is an $\alpha$, $\beta$- unsaturated ketone, in which $R_5$ has the indicated meaning, which is reacted with a substituted hydrazine, the substituent of which has the meaning indicated for $R_4$, in the presence of an excess base, at a temperature in the range from 70 °C to 100 °C, in an inert solvent, and the reaction product is isolated and purified.

7. Use of the compounds of the general formula I as claimed in claim 1, as photoconductive substances.

**Revendications**

1. Dérivés de type hydrazine du 4-chloro-oxazole de formule générale I

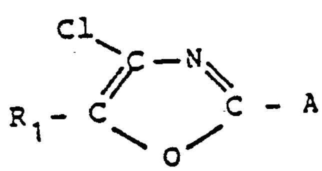

(I)

dans laquelle A possède l'une des structures suivantes:

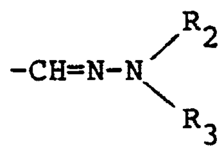

ou

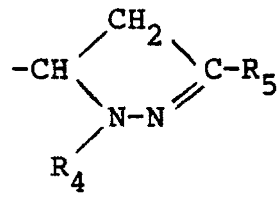

et

R1 représente un groupe phényle qui peut être substitué une ou plusieurs fois par un ou des atomes d'halogène ou radicaux alcoxy en $C_1$-$C_4$,

R2, R3 représentent un groupe alkyle en $C_1$-$C_6$ ou phényle qui peut être substitué par des atomes d'halogène ou radicaux alcoxy en $C_1$-$C_4$, ou

R2 et R3 forment ensemble un groupement $-(CH_2)_n-$ avec n = 4 à 6, et

R4 représente un groupe phényle qui peut être substitué une ou plusieurs fois par un ou des atomes d'halogène ou radicaux alcoxy en $C_1$-$C_4$, et

R5 représente un groupe phényle qui peut être substitué une ou plusieurs fois par un ou des radicaux alcoxy en $C_1$-$C_4$,dialkyl($C_1$-$C_4$)-amino, pyrrolidine-1-yle, pipéridine-1-yle ou morpholine-4-yle.

**2.** Composés selon la revendication 1, dans lesquels

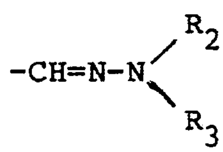

A représente

R1 représente un groupe alcoxy($C_1$-$C_4$)-phényle et

R2 et R3 représentent le groupe phényle.

**3.** Composés selon la revendication 1, dans lesquels

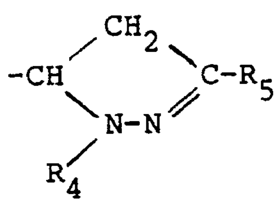

A représente

R1 représente un groupe phényle qui peut être substitué par des radicaux alcoxy en $C_1$-$C_4$, et

R4 et R5 représentent le groupe phényle.

**4.** Procédé pour la préparation de dérivés de type hydrazine du 4-chloro-oxazole selon la revendication 1, caractérisé en ce que l'on fait réagir avec une hydrazine substituée un dérivé du 4-chloro-oxazole contenant des groupes carbonyle, dans un solvant inerte, en présence d'une base en excès, à des températures dans la plage d'environ 60 à 100 °C, puis on isole et purifie le produit de réaction.

**5.** Procédé selon la revendication 4, caractérisé en ce que le dérivé du 4-chloro-oxazole contenant des groupes carbonyle est un 4-chloro-oxazole-2-carbaldéhyde, dans lequel R1 a la signification indiquée, que l'on fait réagir avec une hydrazine dans laquelle R2 et R3 ont les significations indiquées, en présence d'une base en excès, à une température dans la plage de 60 à 80 °C, dans un solvant inerte, et on isole et purifie le produit de réaction.

6. Procédé selon la revendication 4, caractérisé en ce que le dérivé du 4-chloro-oxazole contenant des groupes carbonyle est une cétone $\alpha,\beta$-insaturée, dans laquelle $R_5$ a la signification indiquée, que l'on fait réagir avec une hydrazine substituée dont le substituant a la signification indiquée pour $R_4$, en présence d'une base en excès, à une température dans la plage de 70 à 100 °C, dans un solvant inerte, et on isole et purifie le produit de réaction.

7. Utilisation des composés de formule générale I selon la revendication 1, en tant que substances photoconductrices.